# FASCICULE DE BREVET EUROPEEN

(11) **EP 2 403 955 B1**
(45) Date de publication et mention de la délivrance du brevet: **19.10.2016**
(21) Numéro de dépôt: 10710080.2
(22) Date de dépôt: 24.02.2010
(51) Int. Cl.: C12P 7/64, C11C 1/08, C11C 3/04

(54) **PROCÉDÉ DE PRODUCTION D'ESTER D'ACIDE RICINOLÉIQUE PAR TRANSESTÉRIFICATION ENZYMATIQUE SÉLECTIVE**
VERFAHREN ZUR HERSTELLUNG VON RICINOLSÄUREESTER DURCH SELEKTIVE ENZYMATISCHE TRANSESTERIFIZIERUNG
METHOD FOR PRODUCING RICINOLEIC ACID ESTER BY SELECTIVE ENZYMATIC TRANSESTERIFICATION

(30) Priorité: 02.03.2009 FR 0951302
(43) Date de publication de la demande: 11.01.2012
(73) Titulaire: Arkema France, 92700 Colombes (FR)
(72) Inventeur: DUBOIS, Jean-Luc, F-69390 Millery (FR); BOURLIEU-LACANAL, Claire, F-35000 Rennes (FR); LECOMTE, Jérôme, F-34980 St Gely Du Fesc (FR); DUBREUCQ, Eric, F-34000 Montpellier (FR); VILLENEUVE, Pierre, F-34090 Montpellier (FR)
(86) Numéro de dépôt international: PCT/FR2010/050314
(87) Numéro de publication internationale: WO 2010/100366

(56) Documents cités:
- FR-A1- 2 921 364
- FOGLIA T A ET AL: "SELECTIVITY OF LIPASES: ISOLATION OF FATTY ACIDS FROM CASTOR, CORIANDER, AND MEADOWFOAM OILS", EUROPEAN JOURNAL OF LIPID SCIENCE AND TECHNOLOGY, WILEY VCH VERLAG, WEINHEIM, DE, vol. 102, no. 10, 1 octobre 2000 (2000-10-01), pages 612-617, XP000959999, ISSN: 1438-7697
- NAUGHTON F C: "PRODUCTION CHEMISTRY AND COMMERCIAL APPLICATIONS OF VARIOUS CHEMICALS FROM CASTOR OIL", JOURNAL OF THE AMERICAN OIL CHEMISTS' SOCIETY, SPRINGER, BERLIN, DE, vol. 51, no. 3, 1 janvier 1974 (1974-01-01), pages 65-71, XP002469296, ISSN: 0003-021X
- BREIVIK H ET AL: "PREPARATION OF HIGHLY PURIFIED CONCENTRATES OF EICOSAPENTAENOIC ACID AND DOCOSAHYXAENOIC ACID", JOURNAL OF THE AMERICAN OIL CHEMISTS' SOCIETY, SPRINGER, BERLIN, DE, vol. 74, no. 11, 1 janvier 1997 (1997-01-01), pages 1425-1429, XP002946598, ISSN: 0003-021X
- HAYES D G: "ENZYME-CATALYZED MODIFICATION OF OILSEED MATERIALS TO PRODUCE ECO-FRIENDLY PRODUCTS", JOURNAL OF THE AMERICAN OIL CHEMISTS' SOCIETY, SPRINGER, BERLIN, DE, vol. 81, no. 12, 1 décembre 2004 (2004-12-01), pages 1077-1103, XP001221074, ISSN: 0003-021X
- BERDEAUX O ET AL: "LARGE-SCALE SYNTHESIS OF METHYL CIS-9,TRANS-11-OCTADECADIENOATE FROM METHYL RICINOLEATE", JOURNAL OF THE AMERICAN OIL CHEMISTS' SOCIETY (JAOCS), SPRINGER, DE, vol. 74, no. 8, 1 January 1997 (1997-01-01), pages 1011-1015, XP002485889, ISSN: 0003-021X, DOI: 10.1007/S11746-997-0018-Z

## Description

La présente invention se rapporte d'une manière générale au domaine de l'oléochimie. Plus particulièrement, l'invention concerne un procédé de préparation d'ester d'acide ricinoléique à partir d'une huile végétale pure ou en mélange, notamment l'huile de ricin, ledit procédé comprenant au moins une étape de transestérification enzymatique réalisée en présence d'un alcohol aliphatique inférieur et au moyen d'une lipase typosélective, et conduisant à l'obtention d'une fraction enrichie en ester d'acide ricinoléique.

Les esters d'acides gras sont classiquement obtenus par transestérification chimique de l'huile végétale raffinée, en présence d'un catalyseur, notamment acide ou basique. Cette voie de production d'esters d'acides gras permet d'obtenir lesdits esters avec de bons rendements ; elle présente toutefois de nombreux inconvénients, liés notamment au besoin d'éliminer le catalyseur utilisé, à la récupération difficile du glycérol et à la forte consommation d'énergie. Cet ensemble de procédés demeure laborieux, car comportant de nombreuses étapes de transformation physico-chimiques, ce qui conduit à un coût élevé des esters d'acides gras ainsi fabriqués. De plus, les catalyseurs acides ou basiques utilisés ne sont pas sélectifs quant au type d'acide gras, et transestérifient toutes les chaînes d'acides gras, conduisant à l'obtention d'un mélange d'esters. Or, il est très difficile de séparer les différents esters d'acides gras, qui présentent des propriétés physiques relativement proches. En outre, ce type de procédé ne permet pas d'extraire sélectivement les esters d'acides gras fonctionnalisés, notamment hydroxylés, du mélange d'esters d'acides gras obtenus par la réaction de transestérification. Or, il est souhaitable de disposer d'une fraction enrichie en esters d'acides gras hydroxylés, contenant par exemple du ricinoléate de méthyle (ou 12-hydroxy cis 9-octadécénoate de méthyle), car une telle fraction représente le point de départ dans la fabrication d'acide 11-amino undécanoïque, monomère constitutif du Rilsan^{®}11, qui est un polyamide aux propriétés physiques exceptionnelles, développé par la demanderesse. Lors de la fabrication de l'acide 11-amino undécanoïque, le ricinoléate de méthyle est amené à subir un craquage thermique en phase gazeuse. A cet effet, il doit contenir un minimum de glycérides, c'est à dire de tri-, di- et monoglycérides car ces produits sont très difficiles à vaporiser, et souvent se décomposent avant vaporisation, ce qui a pour conséquence de baisser la sélectivité du craquage. De même, le ricinoléate de méthyle doit contenir un minimum d'acide ricinoléique, lui aussi difficile à vaporiser.

Au vu de ce qui précède, la demanderesse s'est fixé comme but de trouver un procédé de transestérification d'huiles végétales contenant de l'acide ricinoléique, palliant les inconvénients précités des procédés de transestérification connus et permettant d'obtenir une fraction riche en ester d'acide ricinoléique.

Des procédés d'estérification ou hydrolyse des triglycérides contenus dans les huiles végétales en présence de lipases sont bien connus. Ces enzymes présentent plusieurs avantages : elles ne nécessitent pas de cofacteur, sont souvent disponibles dans le commerce et présentent une activité et une sélectivité élevées, même dans des systèmes non aqueux. Les esters de glycérol ou triglycérides sont leur substrat naturel. Certaines de ces lipases présentent une spécificité de position (ou régioséléctivité), qui leur permet de distinguer entre la position centrale (*sn*-2) et les deux positions externes du glycérol (*sn*-1 et *sn*-3)*.* Cette spécificité ne peut toutefois être mise à profit dans le cas de l'acide ricinoléique (AR) présent dans l'huile de ricin qu'au prix d'une perte de rendement, car celle-ci est composée majoritairement des résidus d'AR (de 85 à 90% en poids), les autres résidus d'acides gras présents étant notamment ceux des acides oléique, linoléique, stéarique, palmitique et linolénique. L'acide ricinoléique occupe de préférence les positions externes, mais peut se retrouver également en position interne. Par ailleurs, la littérature ne décrit aucune lipase spécifique de l'AR, dans une réaction de transestérification. Même la lipase extraite de ricin, qui présente une préférence pour l'AR lors de la réaction d'hydrolyse, ne montre aucune typoséléctivité à son égard.

D'autres lipases présentent une typoséléctivité ou spécificité vis-à-vis de la nature de l'acide gras. Il est ainsi connu que la lipase extraite de *Geotrichum candidum,* champignon levuriforme, présente une forte spécificité envers les acides gras monoinsaturés en *cis*-9 dans des réactions d'estérification ou d'hydrolyse. Le document DE 41 24 248 décrit l'utilisation de lipases extracellulaires extraites de *Geotrichum candidum* pour cliver spécifiquement des esters d'acide oléique (CH₃(CH₂)₇CH=CH(CH₂)₇COOH). Le document US 5 633 151 décrit l'utilisation de la même lipase pour cliver spécifiquement des esters d'acide érucique (CH₃(CH₂)₇CH=CH(CH₂)₁₁COOH).

La publication de Foglia T.A. et al., Eur. J Lipid Sci. Technol. 102 (2000): 612-617 décrit les résultats d'essais visant à déterminer la sélectivité de certaines lipases envers des acides gras d'origine végétale. Les résultats présentés dans le tableau 1 montrent que la lipase de *Geotrichum candidum* est discriminante envers l'acide ricinoléique (ci-après désigné par AR), lors de la réaction d'hydrolyse partielle de l'huile de ricin effectuée à 30°C pendant 1 à 4 h avec une lipase immobilisée commerciale. Par ailleurs, les résultats figurant dans le tableau 2 montrent que cette enzyme est également discriminante envers l'AR lors de la réaction d'estérification d'un mélange d'acides gras libres avec le 1-butanol. L'estérification en présence de G. *candidum* conduit, après 24h de réaction, à un mélange constitué (taux massiques) de 12% d'esters butyliques d'acides gras et 88% d'acides gras libres, ces derniers étant représentés à 94,5% par l'AR. Ledit mélange comporte donc environ 83% d'AR.

Le comportement particulier d'une enzyme vis à vis d'un substrat au cours d'une réaction d'hydrolyse ne préjuge pas de son comportement envers le même substrat lors d'une réaction de transestérification ou d'estérification. La spécificité d'une lipase varie d'un type de réaction à un autre. La publication de Vaysse L. et al., Enzyme Microb. Technol. 31 (2002) : 648-655 montre à travers les résultats figurant dans les tableaux 1 et 2 qu'une même lipase peut avoir un comportement différent envers le même ester d'acides gras ou l'acide gras libre lors de la réaction d'hydrolyse, transestérification et estérification respectivement.

La demanderesse a mené des études visant à établir l'effet de la lipase de *Geotrichum candidum* sur les acides gras présents dans l'huile de ricin, notamment sur l'acide ricinoléique (CH₃(CH₂)₅CH(OH)CH₂CH=CH(CH₂)₇COOH) lors de la réaction de transesérification de l'huile de ricin. Ces études ont montré que, d'une manière surprenante, ladite lipase est discriminante envers l'AR dans des réactions de transestérification en présence d'un alcool aliphatique intérieur.

L'objet de la présente invention est de fournir un procédé de transestérification qui permet l'extraction biosélective d'ester d'acide ricinoléique, et donc l'obtention d'une fraction enrichie en cet ester (contenant plus de 85% en poids en ester) à partir de l'huile de ricin ou toute autre huile contenant des résidus d'AR.

A cet effet, l'invention concerne, selon un premier aspect, un procédé de production d'ester d'acide ricinoléique à partir d'une huile végétale contenant de l'acide ricinoléique, notamment l'huile de ricin, comprenant les étapes suivantes :
i) une première réaction de transestérification effectuée en présence d'un alcool aliphatique inférieur choisi parmi le méthanol, l'éthanol, l'isopropanol et le n-propanol, et de la lipase extraite de *Geotrichum candidum,* conduisant à l'obtention d'un mélange M comprenant des mono-, di- et triglycérides d'AR, esters d'acides gras autres que l'AR, alcool ;
ii) une étape de séparation dudit mélange M en au moins deux fractions, dont une fraction A enrichie en glycérides d'AR, et une fraction B riche en esters d'acides gras autres que l'AR ;
iii) une étape de transformation desdits glycérides d'AR en esters d'AR formant une fraction C riche en esters d'AR et une fraction riche en alcools.

Avantageusement, ladite lipase coupe sélectivement la liaison ester impliquant les acides gras autres que l'AR, notamment les acides oléique et linoléique, et laisse quasiment intactes les liaisons acyles impliquant un résidu d'AR.

La mise en oeuvre du procédé selon l'invention permet d'obtenir une fraction C particulièrement riche en ester d'acide ricinoléique. La fraction enrichie en ester d'AR peut contenir au moins 91% en poids, et de préférence de 94 à 98% en poids d'ester méthylique d'acide ricinoléique lorsque la matière première est une huile de ricin pure. Une telle fraction convient très avantageusement à une utilisation comme matière première dans la synthèse de produits chimiques intermédiaires tels que l'acide 11-amino undécanoïque.

Lorsque la matière première est une huile pauvre en acide ricinoléique telle que définie ci-dessous, le procédé selon l'invention permet d'obtenir une fraction enrichie en ester d'AR contenant au moins 75% en poids, et de préférence de 82 à 91% en poids d'ester méthylique d'acide ricinoléique. Une telle fraction convient en particulier à une utilisation comme matière première dans la synthèse de produits chimiques intermédiaires tels que l'acide 11-amino undécanoïque, puisqu'ayant alors une teneur en acide ricinoléique équivalente aux huiles de ricin conventionnelles.

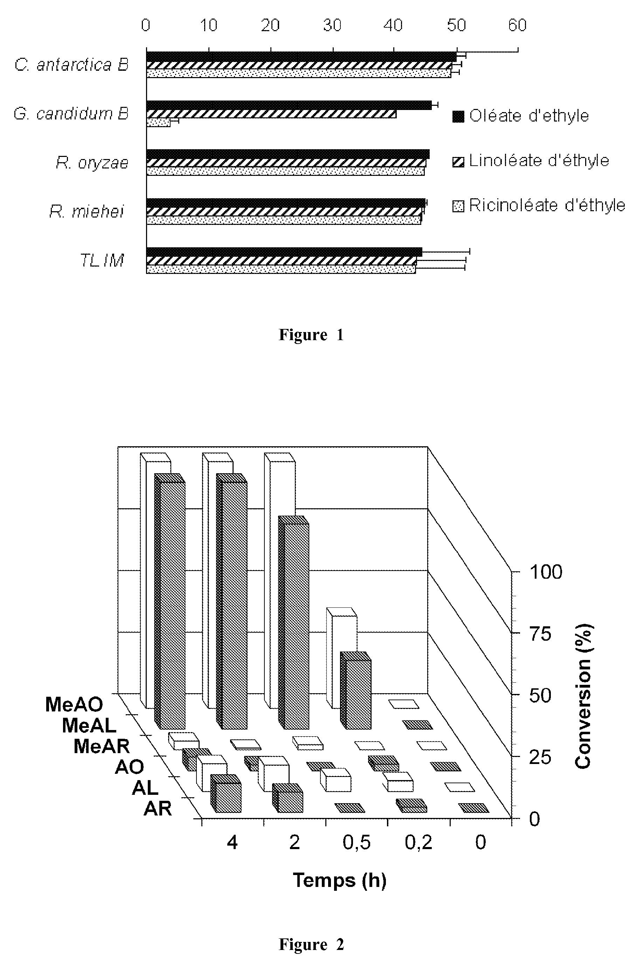

D'autres caractéristiques et avantages ressortiront de la description détaillée du procédé de production d'ester d'acide ricinoléique selon l'invention qui va suivre et des figures 1 à 10 annexées, représentant :
- La figure 1 illustre le taux de conversion d'esters éthyliques en esters méthyliques en réaction de transestérification en présence de méthanol, obtenus avec une lipase typosélective de *Geotrichum candidum* sous forme libre et diverses lipases commerciales ;
- La figure 2 montre les cinétiques de conversion des groupements ricinoléoyl, linoléoyl et oléoyl initialement sous forme de glycérides dans l'huile de ricin en esters méthyliques ou acides gras correspondants pendant la réaction de transestérification en présence de méthanol catalysée par la lipase typosélective de G. *candidum;*

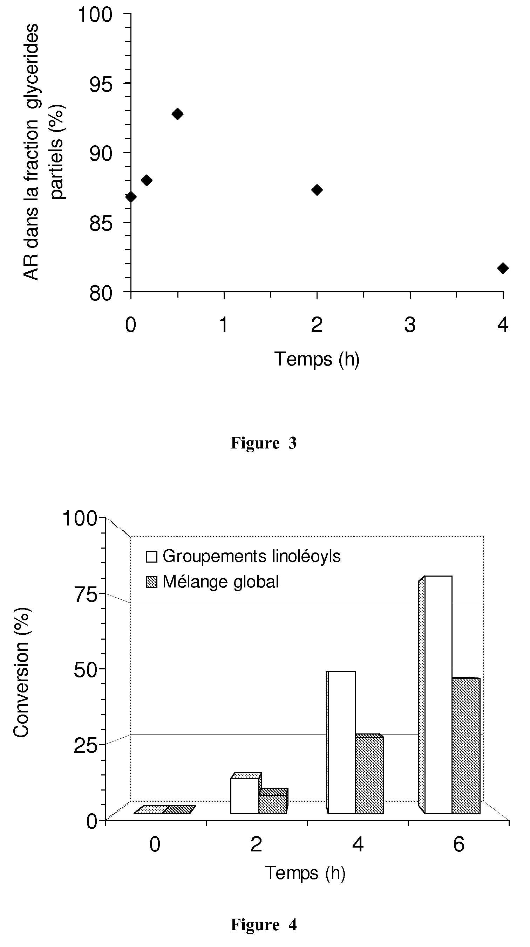

- La figure 3 illustre l'évolution de la concentration en AR dans la fraction glycérides partiels lors de la transestérification de l'huile de ricin en présence de méthanol catalysée par la lipase typosélective de G. *candidum ;*
- La figure 4 montre une comparaison des cinétiques de conversion des groupements linoléoyl et des groupements acyles en esters méthyliques durant une réaction de transestérification en présence de méthanol d'un mélange huile de Ricin/trilinoléine 50:50 p/p (en poids) catalysée par la lipase typosélective *de G. candidum ;*

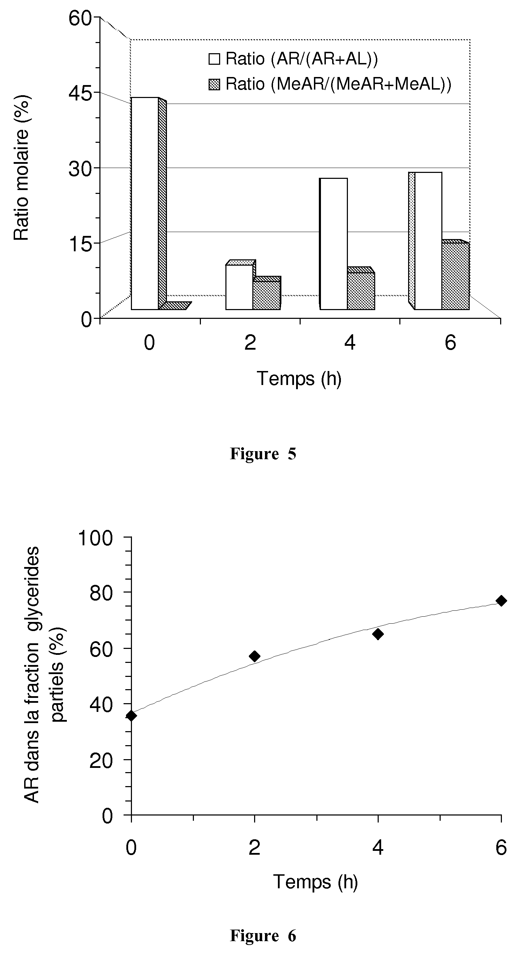

- La figure 5 illustre l'évolution des ratios [AR/(AR+AL)] et [MeAR/(MeAR+MeAL)] représentatifs de la discrimination d'une lipase typosélective de G. *candidum* envers les groupements ricinoléoyl et de sa sélectivité envers les groupements linoléoyls durant une réaction de transestérification en présence de méthanol d'un mélange huile de Ricin/trilinoléine 50:50 p/p catalysée par la lipase typosélective de G. *candidum*
- La figure 6 présente l'évolution de la concentration en AR dans la fraction glycérides partiels lors de la transestérification en présence de méthanol d'un mélange huile de Ricin/trilinoléine 50:50 p/p catalysée par la lipase typosélective de *G. candidum ;*

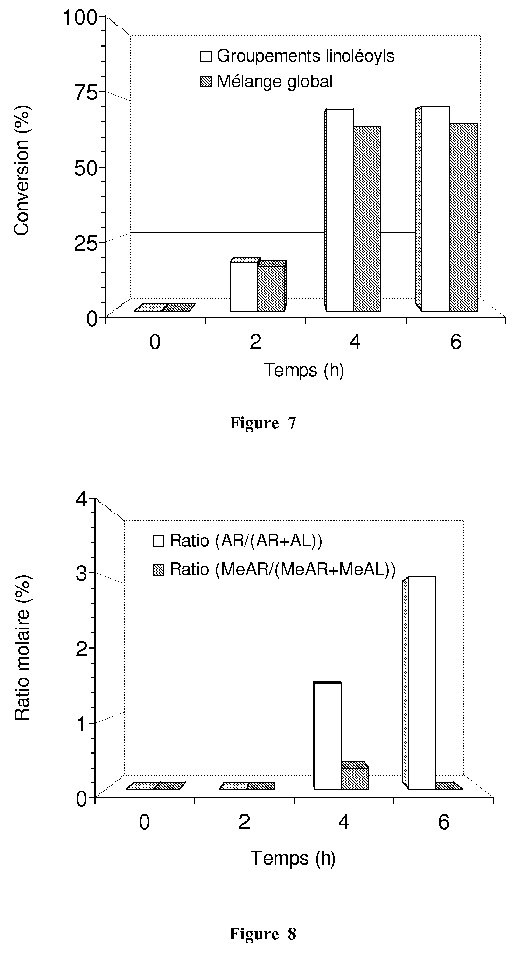

- La figure 7 illustre la comparaison des cinétiques de conversion des groupements linoléoyls et des groupements acyles en esters méthyliques durant une réaction de transestérification en présence de méthanol d'un mélange huile de Ricin/trilinoléine 90:10 p/p catalysée par la lipase typosélective de *G. candidum ;*
- La figure 8 illustre l'évolution des ratios [AR/(AR+AL)] et [MeAR/(MeAR+MeAL)] représentatifs de la discrimination de la lipase typosélective de *G. candidum* envers les groupements ricinoléoyls et de sa sélectivité envers les groupements linoléoyls durant une réaction de transestérification en présence de méthanol d'un mélange huile de Ricin/trilinoléine 90:10 p/p ;

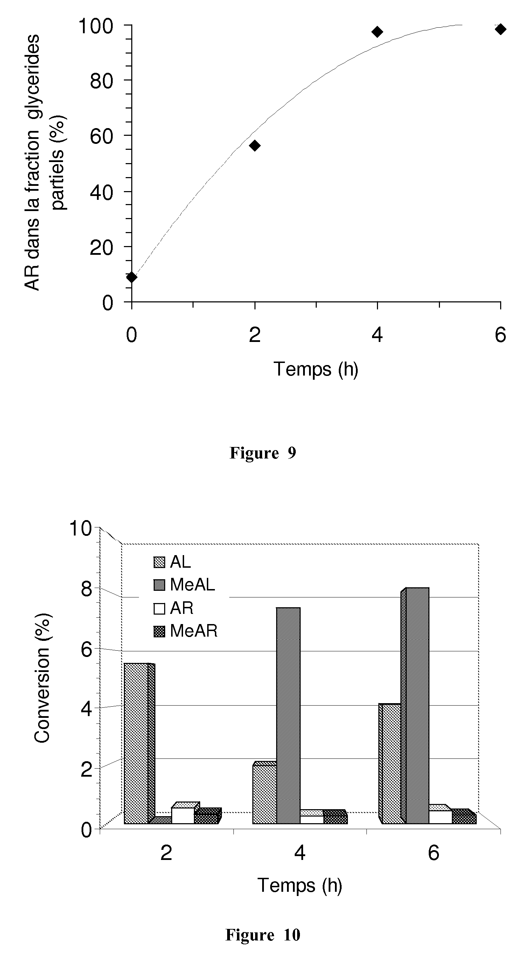

- La figure 9 montre l'évolution de la concentration en AR dans la fraction glycérides partiels lors de la transestérification en présence de méthanol d'un mélange huile de Ricin/trilinoléine 90:10 p/p catalysée par la lipase typosélective de *G. candidum ;*
- La figure 10 présente les cinétiques de conversion des groupements ricinoléoyls et linoléoyls initialement sous forme de glycérides, en esters méthyliques ou acides gras correspondants, pendant la réaction de transestérification d'un mélange huile de Ricin/trilinoléine 50:50 p/p, en présence de méthanol, catalysée par la lipase typosélective de *G. candidum* immobilisée.

La présente invention se rapporte à un procédé de transestérification enzymatique d'une huile végétale contenant de l'acide ricinoléique, couplé à un procédé de transestérification chimique ou enzymatique, dans le but de produire une coupe riche en ester d'acide ricinoléique. De manière caractéristique, le procédé met en oeuvre une enzyme qui présente une « anti »sélectivité (appelée encore discrimination) envers l'acide ricinoléique, c'est à dire qui transestérifie de préférence les chaînes autres que l'acide ricinoléique. Cet effet a notamment été mis en évidence sur les deux principaux acides gras autres que l'acide ricinoléique de l'huile de ricin : l'acide oléique et l'acide linoléique.

Le procédé de transestérification enzymatique de l'invention est particulièrement bien adapté à une huile « pauvre » en acide ricinoléique, c'est à dire contenant moins de 82 % en poids d'acide ricinoléique. Ce cas de figure est celui des huiles de ricin pauvres en acide ricinoléique, ou celui des mélanges comprenant de l'huile de ricin et autres huiles végétales provenant d'au moins une autre plante oléagineuse, oléo-protéagineuse ou protéagineuse, lesdits mélanges contenant au moins 10% en poids d'acide ricinoléique, ou encore celui des huiles provenant des plantes génétiquement modifiées produisant de l'acide ricinoléique, seules ou en mélange avec des plantes oléagineuses éventuellement génétiquement modifiées et contenant au moins 10% en poids d'acide ricinoléique. En référence aux publications de Pilar Rijas-Barros dans Crop Science 44 (2004) : 76-80, et 45 (2005) : 157-162, on comprend aussi par huile de ricin pauvre en acide ricinoléique des huiles issues de mutants comme le OLE-1 décrits dans ces publications et qui produit une huile contenant environ 10 % d'acide ricinoléique. En référence à l'article de K. M. Hosamani dans Chemistry and Physics of Lipids, 152 (2008) 9-12, par huile pauvre en acide ricinoléique on comprend aussi l'huile de l'*Hevea brasiliensis* et du *Jatropha gosypiifolia,* où l'acide ricinoléique a été trouvé à une teneur d'environ 18 %. En référence à un autre article du même auteur, paru dans Phytochemistry, Vol 37, N°6, pp 1621-1624 en 1994**,** on comprend aussi l'huile de *Trichodesma zeylanicum*, qui contiendrait 22 % d'acide ricinoléique. On peut aussi citer le *Mammea africana* qui contient 20 % d'acide ricinoléique dans les graines de ses fruits ainsi que l'huile de graine d'*Alternanthera triandra* Syn. *A. sessilis* qui en contient environ 22% (K. M. Hosamani et al, Industrial Crops and Products, 2004, 19(2),133-136). Par conséquent, l'expression «huile végétale contenant de l'acide ricinoléique » couvre dans le cadre de la présente invention l'huile de ricin ainsi que toutes ces huiles et mélanges d'huiles précités.

Il a maintenant été trouvé qu'une lipase extracellulaire extraite du micoorganisme *Geotrichum candidum* est sélective vis-à-vis des acides gras mono insaturés en position cis-9 autres que l'acide ricinoléique, lors de la réaction de transestérification d'une huile végétale contenant de l'acide ricinoléique, effectuée en présence d'un alcool aliphatique inférieur.

Selon un premier aspect, l'invention a pour objet un procédé de production d'ester d'acide ricinoléique à partir d'une huile végétale contenant de l'acide ricinoléique, notamment l'huile de ricin, comprenant les étapes suivantes :
i) une première réaction de transestérification enzymatique effectuée en présence d'un alcool aliphatique inférieur choisi parmi le méthanol, l'éthanol, l'isopropanol et le n-propanol, et de la lipase typosélective extraite d'une souche de *Geotrichum candidum,* conduisant à l'obtention d'un mélange M comprenant des mono-, di- et triglycérides d'AR, esters d'acides gras autres que l'AR, et l'alcool ;
ii) une étape de séparation dudit mélange M en au moins deux fractions, dont une fraction A enrichie en glycérides d'AR, et une fraction B riche en esters d'acides gras autres que l'AR ;
iii) une étape de transformation desdits glycérides d'AR contenus dans la fraction A en esters d'AR pour former une fraction C riche en esters d'AR et une fraction riche en alcools.

Avantageusement, lors de l'étape i), la durée de la réaction de transestérification est d'au plus 1h, de préférence d'au plus 30 minutes, ce qui correspond à une teneur d'au moins 90% en poids d'AR par rapport aux autres acides gras, dans la fraction de glycérides partiels du mélange M.

Selon un premier mode de réalisation, l'étape iii) du procédé de transestérification consiste en une réaction de transestérification chimique de la phase comprenant des glycérides d'AR et de l'alcool, en présence d'un catalyseur basique, comme la soude, pour obtenir ladite fraction C riche en esters d'AR.

Selon un deuxième mode de réalisation, l'étape iii) consiste en une seconde réaction de transestérification enzymatique effectuée en présence d'un alcool aliphatique inférieur et de lipase par exemple choisie parmi *Candida antarctica B* (notamment sous forme immobilisée N. 435®, Novo Nordisk), *Rhizomucor miehei* (notamment sous forme immobilisée RM® IM, Novozymes*)*, *Thermomyces lanuginosa* (notamment sous forme immobilisée TL® IM, Novozymes*)*, *Pseudomonas cepacia, Aspergillus neiger, Rhizopus oryzae, Rhizopus arrhizus*, *Candida rugosa*, *Mucor javanicus,* lipase pancréatique porcine et toute autre lipase conduisant à l'obtention de ladite fraction C riche en esters d'AR.

L'alcool aliphatique inférieur utilisé est choisi parmi le méthanol, l'éthanol, l'isopropanol et le n-propanol, et de préférence est le méthanol.

Le ratio substrat/alcool dans l'étape i) varie de 1 : 4,5 à 1 : 0,75, l'alcool étant ajouté en une ou en plusieurs fois.

Compte tenu de la composition de l'huile de ricin (voir l'exemple 1), les esters d'acides gras autres que l'AR les plus importants sont les esters d'acide oléique et linoléique. L'étape ii) de séparation du mélange de glycérides de l'acide ricinoléique / esters méthyliques des acides oléiques/linoléiques comprend au moins une opération choisie parmi : distillation, centrifugation, décantation, extraction liquide-liquide au moyen de solvants organiques apolaires tels que par exemple l'hexane ou l'éther de pétrole, séparation chromatographique par adsorption sélective.

*Geotrichum candidum* est un Endomycète de la famille des Dipodascaceae, ayant une morphologie proche des champignons filamenteux. Il est répandu dans la nature et se retrouve dans de nombreux aliments, dont les produits laitiers. C'est aussi un saprophyte du tube digestif de l'homme et des animaux.

Par « lipase extraite de *Geotrichum candidum* » on entend ici aussi bien le mélange brut de lipases extracellulaires extraites de cette levure, selon le protocole décrit par exemple dans le document DE 41 24 248, qu'une enzyme obtenue par expression hétérologue d'un gène de *Geotrichum candidum* codant pour l'une de ces lipases.

Dans le but d'assurer un bon rendement en vue d'une utilisation à l'échelle industrielle, cette enzyme est utilisée de préférence dans le cadre de l'invention sous une forme immobilisée.

Les conditions d'utilisation de la lipase de *Geotrichum candidum* lors de la phase de transestérification, sont les suivantes :
- température comprise dans la gamme 20 à 40°C, et de préférence d'environ 30°C ;
- ratio lipase/substrat supérieur ou égal à 0,5 % ;
- agitation suffisante pour une bonne homogénéisation du milieu réactionnel de type batch, ou un débit suffisant pour un temps de contact optimal (lit catalytique) ;
- pression atmosphérique ou supérieure selon le procédé mis en oeuvre.

Le procédé est mené à température basse et pression proche de la pression atmosphérique. Les temps de séjour sont aussi limités car on ne cherche pas à convertir la totalité des chaînes grasses mais seulement les impuretés présentes (autres acides gras que l'acide ricinoléique).

Dans une variante de réalisation, la fraction C contenant l'ester d'acide ricinoléique issue du procédé selon l'invention peut avantageusement être directement utilisée dans la synthèse d'acide 11-amino undécanoïque. Lorsque l'ester d'acide ricinoléique n'est pas suffisamment pur, une étape de purification supplémentaire peut être nécessaire, avant de le soumettre à la réaction de pyrolyse.

L'acide 11-amino undécanoïque ainsi obtenu est destiné principalement à la synthèse par condensation de polyamide 11 ou Rilsan^{®} 11, suivant l'enchaînement suivant de réactions :
- pyrolyse ou craquage de l'ester notamment méthylique de l'acide ricinoléique, conduisant à l'obtention d'heptanal et d'undécylénate de méthyle ;
- hydrolyse de l'undécylénate de méthyle conduisant à l'obtention d'acide undécylénique ;
- hydrobromuration de l'acide undécylénique conduisant à l'obtention de l'acide 11-bromoundécanoïque, et
- amination de l'acide 11-bromoundécanoïque conduisant à l'obtention de l'acide 11-amino undécanoïque.

Selon une variante de réalisation, la fraction B enrichie en esters d'acides gras autres que l'AR est soumise à une succession de réactions chimiques et/ou physiques conduisant à l'obtention de biodiesel. Les esters d'acides gras peuvent dans ce cas être soumis à des réactions de séparation par différentes techniques séparatives complémentaires, par exemple par distillation, dont la distillation moléculaire, par extraction liquide-liquide (chromatographie à contre courant en milieu méthanol hexane) ou par séparation chromatographique en lit mobile simulé.

L'invention sera mieux comprise à la lecture des exemples de réalisation non limitatifs suivants.

### 1. Composition en acides gras de l'huile de ricin

Un échantillon d'huile de ricin provenant d'Inde a été analysé quant à sa composition en acides gras. Pour cette détermination, l'huile est transformée en esters méthyliques selon une procédure comportant deux étapes successives, une méthanolyse basique suivie d'une estérification acide. Dans la première étape les glycérides (mono-, di- et triglycérides) sont transformés en esters méthyliques par action du méthylate de sodium et les acides gras libres, éventuellement présents, sont convertis en savons. L'ajout d'une solution méthanolique de chlorure d'éthanoyle conduit, lors de la deuxième étape, à la transformation de ces savons en esters méthyliques. Les esters méthyliques ainsi obtenus sont ensuite analysés par chromatographie en phase gazeuse (Agilent 6890 series GC, équipé d'un détecteur à ionisation de flamme - FID). Les séparations sont réalisées sur une colonne SUPELCOWAX 10 (Agilent Technologies, 30 m x 0,32 mm x 0,25 µm, gaz vecteur: hélium à 1 ml/min ; température initiale du four 150°C, programme de température de 5°C/minute jusqu'à 225°C et palier de 7 min à cette température).

Les principaux acides gras identifiés par cette technique sont :
- l'acide ricinoléique 86,0% en poids ;
- l'acide linoléique 5,3% en poids ;
- l'acide oléique 4,1 % en poids ;
- l'acide stéarique 1,5% en poids ;
- l'acide palmitique 1,3% en poids.

### 2. Production de la lipase tvposélective de Geotrichum candidum

Le protocole opératoire décrit dans le document DE 41 24 248 a été utilisé pour obtenir l'extrait brut de lipases extracellulaires issu de cette levure. Par ailleurs, la lipase a également été obtenue par fermentation de la levure *Pichia pastoris* au moyen d'une technologie recombinante. Ainsi, à l'issue de 30 h de fermentation, le surnageant de fermentation contenant la lipase extracellulaire est recueilli. A ce stade, le surnageant est caractérisé par une concentration protéique de 2,46 mg /ml et une activité hydrolytique spécifique mesurée sur oléate d'éthyle (30°C) de 90 U/ml (avec U = 1 µmol d'ester ethylique hydrolysée par min à 30°C, pH 6.5). Le surnageant est ensuite soumis à une série de filtrations visant à le clarifier et le concentrer sans affecter l'activité spécifique de la lipase qu'il contient : i) micro filtrations sur membrane (seuils de coupure 5 µm puis 0,45 µm), ultrafiltration sur membrane visant à concentrer 14 fois le surnageant puis diafiltration contre une solution tampon de phosphate de sodium pH 6.5, 50 mM. Le concentrât obtenu présente une teneur protéique de 18 mg /ml et une activité spécifique de 522 U/ml sur oléate d'éthyle et de 900 U/ml sur huile d'olive (30°C).

### Immobilisation de la lipase

Le mélange de lipases extraites de *Geotrichum candidum* (ou le concentrât obtenu après fermentation selon le protocole décrit ci-dessus) a été immobilisé sur un support choisi parmi :
- polypropylène microporeux, par exemple du type Accurel ® MP 1000, fabriqué par Accurel systems, Membrana GmbH, Obernburg, Allemagne;
- DEAE cellulose ;
- Célite (diatomite).

A titre d'exemple, l'immobilisation de la lipase typosélective de *Geotrichum candidum* sur Accurel ® MP 1000 comprend, dans un premier temps, une adsorption physique, suivie dans un second temps, d'une réticulation en présence de glutaraldéhyde.

### 3. Activité et spécificité de la lipase typosélective de Geotrichum candidum libre sur substrat modèle, dans une réaction de transestérification en présence de méthanol

L'activité de la lipase typosélective de *Geotrichum candidum* libre en réaction de transestérification en présence de méthanol a été comparée à celle d'enzymes commerciales (*C*. *antarctica B,* N® 435, Novo Nordisk ; *R. oryzae,* FAP 15®, Amano ; *R. mieihei*, Lipozyme RL-IM, Novozymes ; *TL-IM,* Lipozyme, Novozymes). Un mélange équimolaire d'esters éthyliques des acides oléique, linoléique et ricinoléique, (oléate d'éthyle, linoléate d'éthyle, ricinoléate d'éthyle) est mis en présence de l'enzyme (ratio massique lipase/substrat : 5 %). Le méthanol est ajouté à un ratio molaire méthanol/substrat de 1,5 :1, puis le mélange est incubé 24 h à 30°C. Les esters méthyliques formés sont quantifiés par chromatographie en phase gazeuse selon la méthode détaillée à l'exemple 1. Les résultats obtenus figurent dans la figure 1 annexée, qui illustre le taux de conversion d'esters éthyliques en esters méthyliques en réaction de transestérification en présence de méthanol, obtenus avec la lipase typosélective de *Geotrichum candidum* libre et différentes lipases commerciales (24h, 30°C, 3 répliques, ratio lipase/substrat = 1,5 % p/p, ratio molaire méthanol/substrat = 1,5:1).
Quelle que soit l'enzyme, des taux de conversion compris entre 40% et 50% sont obtenus pour l'oléate d'éthyle et le linoléate d'éthyle. Concernant le ricinoléate d'éthyle, seule la lipase typosélective *de Geotrichum candidum* conduit à une conversion inférieure à 40%, avec une valeur comprise entre 2% et 5%. Ces résultats indiquent une très forte sélectivité d'action de la lipase typosélective de G. *candidum* envers les groupements oléoyl et linolénoyl et, en corollaire, une discrimination des groupements ricinoléoyls.

### 4. Activité et spécificité de la lipase typosélective de Geotrichum candidum libre sur l'huile de ricin, dans une réaction de transestérification en présence de méthanol

Les réactions de méthanolyse sont conduites à 30°C, à pression atmosphérique, en présence de tamis moléculaires (3 Å Perlform, Ø 2 mm) à hauteur de 10% p/p du substrat (huile de ricin). Le ratio protéine du concentrât de lipase typosélective de G. *candidum* /substrat est ajusté à 1,5 % p/p et un ratio molaire méthanol/triglycérides d'huile de ricin de 4,5 :1 a été établi en trois additions successives à 0, 2 et 4 heures de réactions. L'ensemble du milieu réactionnel est homogénéisé par vortex (10 s). A différents temps des aliquotes sont prélevés du milieu réactionnel, dilués dans l'acétone/acétonitrile (1:1 v/v) avant d'être analysés par les techniques chromatographiques énoncées ci-dessous.

Les cinétiques de réaction de transestérification ont pu être caractérisées par chromatrographie sur couche mince (CCM) et chromatographie liquide haute performance (CLHP). Plus précisément la CCM a permis de visualiser les classes d'espèces lipidiques formées tandis que certaines de ces espèces ont pu être quantifiées par CLHP suite à l'établissement de courbes de calibration.

Les séparations CCM ont été réalisées par dépôt sur des plaques de verre pré-enduite de silice (100 x 200 mm, Si G 60, Merck) d'échantillons dissous à 10 mg /ml dans l'acétone/acétonitrile (50/50 v/v). Les dépôts (10 µl) ont été réalisés avec un déposeur automatique (Linomat IV, CAMAG). Après élution avec une phase mobile constituée d'hexane/ diethyl ether/ acide acétique (40:60:1 v/v), les espèces présentes ont été révélées par pulvérisation avec une solution de sulfate de cuivre saturée et d'acide phosphorique (50:50 v/v) et incubation (10 minutes, 180°C). Les facteurs de rétention présentés dans le tableau I ci-dessous ont été déterminés par comparaison avec des composés standards.

**Tableau I**

| **Classes de lipides** | **Facteur de retention*** |
|---|---|
| Monoricinoléine | 0,02 |
| Monoglycérides non hydroxylés | 0,07 |
| Diricinoléine | 0,13 |
| Diglycérides monohydroxylés (1,2) | 0,17 |
| Diglycérides monohydroxylés (1,3) | 0,22 |
| Triricinoléine (RRR) | 0,36 |
| Acide ricinoléique (AR) | 0,42 |
| Triglycérides dihydroxylés | 0,47 |
| Méthyl ricinoléate | 0,58 |
| Acides gras non hydroxylés | 0,65 |
| Triglycérides monohydroxylés | 0,69 |
| Autres esters | 0,95 |

| | |
|---|---|
| *Facteur de rétention = distance de migration de l'espèce / distance de migration du front du solvant | |

La séparation des produits par CLHP a été effectuée en utilisant un système automatisé (Thermo-Finningan, Courtaboeuf, France) basé sur une pompe (P1000 XR), un passeur d'échantillons (AS 1000) et un détecteur à diffusion de la lumière (Alltech 500 ELSD).

Deux colonnes Interchim C18 columns (5 µm; 4,6 x 250 mm, Modulo Cart QS Lichrosphere 500 DS 2) montées en série ont servi à la séparation. Les échantillons (1 mg/ml) ont été élués selon un gradient d'un mélange d'acétone/acétonitrile/acide formique (70:30:1 v/v ; X) et de chloroforme (Y). L'élution a été conduite à un flux de 1 ml/min suivant le gradient linéaire suivant : 0 min 100 % X, 30 min 90 % X, 50 min 90 % X, retour aux conditions initiales en 5 min et maintien de ces conditions pour 15 min supplémentaires.

Les temps de rétention des principales espèces analysées par HPLC suivant le protocole décrit ci-dessus figurent dans le Tableau II ci-après.

Les produits obtenus lors de la transestérification en présence de méthanol sont essentiellement les acides ricinoléique (AR), linoléique (AL), oléique (AO) et les esters méthyliques correspondant, ricinoléate de méthyle (MeAR), linoléate de méthyle (MeAL) et oléate de méthyle (MeAO).

**Tableau II**

| **Espèces analysées** | **Temps de rétention (min)** |
|---|---|
| Acide ricinoléique | 7,2 |
| Ricinoléate de méthyle | 8,1 |
| Monoricinoléine | 9,3 |
| Acide linoléique | 9,5 |
| Diricinoléine | 11,5 |
| Linoléate de méthyle | 11,8 |
| Acide oléique | 12,3 |
| Oléate de méthyle | 14,6 |
| Triricinoléine | 19,8 |
| Trilinoléine | 22,6 |

Les cinétiques de conversion des groupements ricinoléoyls, linoléoyls et oléoyls, initialement présents sous forme de glycérides dans l'huile de Ricin, en esters méthyliques ou acides gras correspondants pendant la réaction de transestérification en présence de méthanol catalysée par la lipase typosélective de G. candidum (30°C, 3 répliques, ratio protéines extrait lipasique/substrat = 1,5 % p/p, ratio molaire méthanol/triglycérides = 4,5:1 en 3 additions fractionnées à t=0, 2 et 4 h) sont illustrées dans la figure 2.

La conversion des groupements ricinoléoyls, linoléoyls et oléoyls (initialement estérifiés dans l'huile de Ricin) en acides gras et esters méthyliques conduit à l'apparition d'une fraction glycérides partiels dont la teneur en AR est maximale entre 0,5 h et 2 h de réaction. La Figure 3 annexée illustre l'évolution de la concentration en AR dans la fraction glycérides partiels lors de la transestérification de l'huile de ricin en présence de méthanol catalysée par la lipase typosélective de G. candidum (30°C, 3 répliques, ratio protéines extrait lipasique/substrat = 1,5 % p/p, ratio molaire méthanol/triglycérides = 4,5:1 en 3 additions fractionnées à t=0, 2 et 4 h).

### 5. Activité et spécificité de la lipase typosélective de Geotrichum candidum libre sur l'huile de ricin en mélange, dans une réaction de transestérification en présence de méthanol

Dans tous les exemples ci-dessous, les réactions de méthanolyse ont été conduites dans des conditions similaires à celles énoncées à l'exemple 3.

### 5.1 huile de ricin/trilinoléine 50/50 p/p

La figure 4 annexée montre une comparaison des cinétiques de conversion des groupements linoléoyls et des groupements acyles en esters méthyliques durant une réaction de transestérification en présence de méthanol d'un mélange huile de ricin/trilinoléine 50:50 p/p catalysée par la lipase typosélective de *G. candidum* (30°C, 3 répliques, ratio protéines extrait lipasique/substrat = 1,5 % p/p, un ratio molaire méthanol/triglycérides de 4,5:1 en 3 additions fractionnées à t égal à 0, 2 et 4 h). Un taux de conversion de 80 % des groupements linoléoyls initialement estérifiés dans le mélange d'huile est atteint en 6 heures de réaction.

Le ratio groupements ricinoléoyls sur l'ensemble des groupements ricinoléoyls et linoléoyls dans les produits de réaction est caractéristique de la discrimination de la lipase envers le groupement ricinoléoyl et à l'inverse de sa sélectivité envers le groupement linoléoyl.

En parallèle à cette réaction de méthanolyse, les molécules d'eau apportées par le concentrât lipasique entraînent la formation d'acide gras libres (AR, AL) par réaction d'hydrolyse. Cette discrimination est plus forte en réaction de méthanolyse qu'en réaction d'hydrolyse, comme représenté sur la figure 5 annexée. Cette figure illustre l'évolution des ratios [AR/(AR+AL)] et [MeAR/(MeAR+MeAL)] représentatifs de la discrimination de la lipase typosélective de *G. candidum* envers les groupements ricinoléoyls et de sa sélectivité envers les groupements linoléoyls durant une réaction de transestérification en présence de méthanol d'un mélange huile de Ricin/trilinoléine 50:50 p/p catalysée par la lipase typosélective de *G. candidum* (30°C, 3 répliques, un ratio protéines extrait lipasique/substrat de 1,5 % p/p, un ratio molaire méthanol/triglycérides de 4,5:1 en 3 additions fractionnées à t égal à 0, 2 et 4 h).

Finalement, cette discrimination entraîne l'enrichissement d'une concentration initiale en AR de 35 % (AR sous forme de glycérides dans le mélange d'huile de départ) à plus de 77 % dans la fraction glycérides partiels du milieu réactionnel après 6 h de réaction, comme représenté sur la figure 6 annexée, qui illustre l'évolution de la concentration en AR dans la fraction glycérides partiels lors de la transestérification en présence de méthanol d'un mélange huile de Ricin/trilinoléine 50:50 p/p catalysée par la lipase typosélective de *G. candidum* (30°C, 3 répliques, un ratio protéines extrait lipasique/substrat de 1,5 % p/p, un ratio molaire méthanol/triglycérides de 4,5:1 en 3 additions fractionnées à t égal à 0, 2 et 4 h).

### 5.2 huile de ricin/trilinoléine 10/90 p/p

Les cinétiques de conversion des groupements linoléoyls et des groupements acyles en esters méthyliques durant une réaction de transestérification en présence de méthanol d'un mélange huile de Ricin/trilinoléine 90:10 p/p catalysée par la lipase typosélective de *G. candidum* (30°C, 3 répliques, un ratio protéines extrait lipasique/substrat de 1,5 % p/p, un ratio molaire méthanol/triglycérides de 4,5:1 en 3 additions fractionnées à t égal à 0, 2 et 4 h), ont été comparées et les résultats obtenus sont montrés dans la figure 7 annexée.

La Figure 8 montre l'évolution des ratios [AR/(AR+AL)] et [MeAR/(MeAR+MeAL)] représentatifs de la discrimination de la lipase typosélective de *G. candidum* envers les groupements ricinoléoyls et de sa sélectivité envers les groupements linoléoyls durant une réaction de transestérification en présence de méthanol d'un mélange huile de Ricin/trilinoléine 90:10 p/p (30°C, 3 répliques, un ratio protéines extrait lipasique/substrat de1,5 % p/p, un ratio molaire méthanol/triglycérides de 4,5:1 en 3 additions fractionnées à t égal à 0, 2 et 4 h).

La figure 9 montre l'évolution de la concentration en AR dans la fraction glycérides partiels lors de la transestérification en présence de méthanol d'un mélange huile de Ricin/trilinoléine 90:10 p/p catalysée par la lipase typosélective de *G. candidum* (30°C, 3 répliques, un ratio protéines extrait lipasique/substrat de 1,5 % p/p, un ratio molaire méthanol/triglycérides de 4,5:1 en 3 additions fractionnées à t égal à 0, 2 et 4 h).

### 5.3 huile de ricin/huile de lin 50/50 p/p

L'huile de lin utilisée en mélange avec l'huile de ricin est composée de glycérides comportant majoritairement des groupements linolénoyls (45 %), linoléoyls (25 %) et oléoyls (19%). Dans des conditions réactionnelles similaires à celles énoncées dans l'exemple 3, un taux de conversion global de ces groupements acyles en esters méthyliques de 17 % est atteint au bout de deux heures. Le taux de ricinoléate de méthyle (MeAR) dans la fraction esters méthyliques est alors de 6 %. Ces résultats indiquent une sélectivité de la lipase typosélective de *G. candidum* envers les groupements linolénoyls similaire à celle envers les groupements linoléoyls.

### 5.4 huile de ricin/huile de lin 10/90 p/p

Dans des conditions réactionnelles similaires à celles énoncées à l'exemple 3, un taux de conversion global de ces groupements acyles en esters méthyliques de 12 % est atteint au bout de deux heures. Le taux de de ricinoléate de méthyle (MeAR) dans la fraction esters méthyliques est alors de 2 %.

### 6. Activité et spécificité de la lipase typosélective de Geotrichum candidum immobilisée sur l'huile de ricin en mélange, dans une réaction de transestérification en présence de méthanol

La lipase typosélective de *Geotrichum candidum* immobilisée sur support Accurel ® MP 1000 a été testée en réaction de transestérification en présence de méthanol sur un mélange huile de Ricin/trilinoléine 50:50 p/p (30°C, 3 répliques, un ratio enzyme/substrat de 1,5 % p/p, un ratio molaire méthanol/triglycérides de 1,5:1). La Figure 10 illustre les cinétiques de conversion des groupements ricinoléoyls et linoléoyls initialement sous forme de glycérides, en esters méthyliques ou acides gras correspondants, pendant la réaction de transestérification d'un mélange huile de Ricin/trilinoléine 50:50 p/p, en présence de méthanol, catalysée par la lipase typosélective de *G*. *candidum* immobilisée (30°C, 3 répliques, un ratio enzyme/substrat de 1,5 % p/p, un ratio molaire méthanol/triglycérides de 1,5:1). Ces résultats montrent que la lipase immobilisée conserve sa très forte sélectivité envers les groupements linoléoyls malgré une baisse significative de son activité. L'hydratation de l'enzyme immobilisée (a_{w} = 0,96) conduit à des réactions d'hydrolyse et l'apparition d'acides gras libres aux cotés des esters méthyliques.

## Revendications

1. Procédé de production d'ester d'acide ricinoléique (AR) à partir d'une huile végétale comprenant de l'acide ricinoléique, comprenant les étapes suivantes :
i) une première réaction de transestérification enzymatique effectuée en présence d'un alcool aliphatique inférieur choisi parmi le méthanol, l'éthanol, l'isopropanol et le n-propanol, et de la lipase extraite de *Geotrichum candidum,* conduisant à l'obtention d'un mélange M comprenant des mono-, di- et triglycérides d'AR, esters d'acides gras autres que l'AR, et l'alcool ;
ii) une étape de séparation dudit mélange M en au moins deux fractions, dont une fraction A enrichie en glycérides d'AR, et une fraction B riche en esters d'acides gras autres que l'AR ;
iii) une étape de transformation desdits glycérides d'AR contenus dans la fraction A en esters d'AR pour former une fraction C riche en esters d'AR et une fraction riche en alcools.

2. Procédé selon la revendication 1 dans lequel l'étape iii) consiste en une réaction de transestérification chimique de la phase comprenant des glycérides d'AR et de l'alcool, en présence d'un catalyseur basique, pour obtenir ladite fraction C riche en esters d'AR.

3. Procédé selon la revendication 1 dans lequel iii) consiste en une seconde réaction de transestérification enzymatique conduisant à l'obtention de ladite fraction C riche en esters d'AR et effectuée en présence d'un alcool aliphatique inférieur et d'une lipase choisie parmi :
- les lipases extraites de : *Candida antarctica B, Rhizomucor miehei*, *Thermomyces lanuginosa, Pseudomonas cepacia, Aspergillus neiger, Rhizopus oryzae*, *Rhizopus arrhizus, Candida rugosa*, *Mucor javanicus ;*
- et la lipase pancréatique porcine.

4. Procédé selon l'une des revendications 1-3 dans lequel ladite lipase typosélective extraite de *Geotrichum candidum* est immobilisée sur un support inorganique ou organique, de nature cellulosique ou un polymère synthétique.

5. Procédé selon l'une des revendications 1 à 4 dans lequel le temps de réaction de l'étape i) est d'au plus 1h.

6. Procédé selon l'une des revendications 1 à 5 dans lequel la température de réaction lors de l'utilisation de ladite lipase extraite de *Geotrichum candidum* est comprise dans la gamme 20 à 40°C.

7. Procédé selon l'une des revendications 1 à 6 dans lequel le ratio lipase/substrat dans l'étape i) est d'au moins 0,5 % p/p.

8. Procédé selon l'une des revendications 1 à 7 dans lequel le ratio substrat/alcool dans l'étape i) varie de 1 : 4,5 à 1 : 0,75, l'alcool étant ajouté en une ou en plusieurs fois.

9. Procédé selon l'une des revendications 1 à 8 dans lequel ledit alcool aliphatique inférieur est le methanol.

10. Procédé selon l'une des revendications 1 à 9 dans lequel l'étape ii) de séparation du mélange M comprend au moins une opération choisie parmi : distillation, centrifugation, décantation, extraction liquide-liquide au moyen de solvants organiques apolaires tels que l'hexane ou l'éther de pétrole, séparation chromatographique par adsorption sélective.

11. Procédé selon l'une des revendications 1 à 10 dans lequel ladite fraction C enrichie en esters d'AR est soumise à une succession de réactions chimiques conduisant à l'obtention d'acide 11-amino undécanoïque.

12. Procédé selon l'une des revendications 1 à 10 dans lequel ladite fraction B enrichie en esters d'acides gras autres que l'AR est soumise à une succession de réactions chimiques et/ou physiques conduisant à l'obtention de biodiesel.

13. Procédé selon l'une des revendications 1 à 10 dans lequel la matière première étant une huile pauvre en acide ricinoléique, ladite fraction enrichie en ester d'AR contient au moins 75% en poids d'ester méthylique d'acide ricinoléique.

14. Procédé de production d'acide 11-aminoundécanoïque, comprenant un procédé de production d'ester d'acide ricinoléique selon la revendication 13 comme première étape.

## Patentansprüche

1. Verfahren zur Produktion von Ricinolsäureester (RS-Ester), ausgehend von einem Pflanzenöl, das Ricinolsäure umfasst, umfassend die folgenden Schritte:
i) eine erste enzymatische Umesterung, die in Gegenwart eines niederen aliphatischen Alkohols, ausgewählt aus Methanol, Ethanol, Isopropanol und n-Propanol, und der aus *Geotrichum candidum* extrahierten Lipase durchgeführt wird, was zur Gewinnung einer Mischung M, umfassend Mono-, Di- und Triglyceride der RS, Ester von Fettsäuren, bei denen es sich nicht um RS handelt, und Alkohol, führt;
ii) einen Schritt der Trennung der Mischung M in mindestens zwei Fraktionen, von denen eine Fraktion A an RS-Glyceriden angereichert ist und eine Fraktion B an Estern von Fettsäuren, bei denen es sich nicht um RS handelt, reich ist;
iii) einen Schritt der Umwandlung der RS-Glyceride, die in der Fraktion A enthalten sind, in RS-Ester unter Bildung einer Fraktion C, die reich an RS-Estern ist, und einer Fraktion, die reich an Alkoholen ist.

2. Verfahren nach Anspruch 1, in dem Schritt iii) aus einer chemischen Umesterung der Phase, die RS-Glyceride und Alkohol umfasst, in Gegenwart eines basischen Katalysators, wodurch man zu der Fraktion C, die reich an RS-Estern ist, gelangt, besteht.

3. Verfahren nach Anspruch 1, in dem Schritt iii) aus einer zweiten enzymatischen Umesterung, die zur Gewinnung der Fraktion C, die reich an RS-Estern ist, führt und die in Gegenwart eines niederen aliphatischen Alkohols und einer Lipase, ausgewählt aus:
- den aus: *Candida antarctica B, Rhizomucor miehei, Thermomyces lanuginosa, Pseudomonas cepacia, Aspergillus niger, Rhizopus oryzae, Rhizopus arrhizus, Candida rugosa, Mucor javanicus* extrahierten Lipasen;
- und Schweinepankreaslipase durchgeführt wird, besteht.

4. Verfahren nach einem der Ansprüche 1-3, in dem die aus *Geotrichum candidum* extrahierte typselektive Lipase auf einem anorganischen oder organischen Träger des Cellulosetyps oder einem synthetischen Polymer immobilisiert ist.

5. Verfahren nach einem der Ansprüche 1 bis 4, in dem die Reaktionsdauer von Schritt i) höchstens 1 h beträgt.

6. Verfahren nach einem der Ansprüche 1 bis 5, in dem die Reaktionstemperatur beim Einsatz der aus *Geotrichum candidum* extrahierten Lipase im Bereich von 20 bis 40°C liegt.

7. Verfahren nach einem der Ansprüche 1 bis 6, in dem das Lipase/Substrat-Verhältnis im Schritt i) mindestens 0,5% w/w beträgt.

8. Verfahren nach einem der Ansprüche 1 bis 7, in dem das Substrat/Alkoholverhältnis in Schritt i) von 1:4,5 bis 1:0,75 schwankt, wobei der Alkohol auf einmal oder in mehreren Portionen zugegeben wird.

9. Verfahren nach einem der Ansprüche 1 bis 8, in dem der niedere aliphatische Alkohol Methanol ist.

10. Verfahren nach einem der Ansprüche 1 bis 9, in dem Schritt ii) der Trennung der Mischung M mindestens einen Vorgang, ausgewählt aus Destillation, Zentrifugation, Dekantieren, Flüssig-Flüssig-Extraktion mit unpolaren organischen Lösungsmitteln wie Hexan oder Petroleumether, chromatographischer Trennung mittels selektiver Adsorption, umfasst.

11. Verfahren nach einem der Ansprüche 1 bis 10, in dem die Fraktion C, die an RS-Estern angereichert ist, einer Folge von chemischen Reaktionen unterworfen wird, die zur Gewinnung von 11-Aminoundecansäure führen.

12. Verfahren nach einem der Ansprüche 1 bis 10, in dem die Fraktion B, die an Estern von Fettsäuren, bei denen es sich nicht um RS handelt, angereichert ist, einer Folge von chemischen und/oder physikalischen Reaktionen unterworfen wird, die zur Gewinnung von Biodiesel führen.

13. Verfahren nach einem der Ansprüche 1 bis 10, in dem, wenn das Ausgangsmaterial ein Öl mit einem niedrigen Gehalt an Ricinolsäure ist, die an RS-Ester angereicherte Fraktion mindestens 75 Gew.-% Ricinolsäuremethylester enthält.

14. Verfahren zur Produktion von 11-Aminoundecansäure, umfassend ein Verfahren zur Produktion von Ricinolsäureester nach Anspruch 13 als ersten Schritt.

## Claims

1. Method for producing ricinoleic acid (RA) ester from a vegetable oil comprising ricinoleic acid, comprising the following steps:
i) a first enzymatic transesterification reaction carried out in the presence of a lower aliphatic alcohol chosen from methanol, ethanol, isopropanol and n-propanol, and of the lipase extracted from *Geotrichum candidum,* producing a mixture M comprising RA mono-, di- and triglycerides, esters of fatty acids other than RA, and alcohol;
ii) a step of separating said mixture M into at least two fractions, including a fraction A enriched with RA glycerides, and a fraction B rich in esters of fatty acids other than RA;
iii) a step of converting said RA glycerides contained in the fraction A into RA esters so as to form a fraction C rich in RA esters and a fraction rich in alcohols.

2. Method according to Claim 1, in which step iii) consists of a chemical transesterification reaction carried out on the phase comprising RA glycerides and alcohol, in the presence of a basic catalyst, so as to obtain said fraction C rich in RA esters.

3. Method according to Claim 1, in which step iii) consists of a second enzymatic transesterification reaction, producing said fraction C rich in RA esters and carried out in the presence of a lower aliphatic alcohol and of a lipase chosen from:
- lipases extracted from: *Candida antarctica B, Rhizomucor miehei, Thermomyces lanuginosa, Pseudomonas cepacia, Aspergillus niger, Rhizopus oryzae, Rhizopus arrhizus, Candida rugosa, Mucor javanicus;*
- and porcine pancreatic lipase.

4. Method according to one of Claims 1 to 3, in which said typoselective lipase extracted from *Geotrichum candidum* is immobilized on a support which is inorganic or organic, of cellulosic nature, or a synthetic polymer.

5. Method according to one of Claims 1 to 4, in which the reaction time in step i) is at most 1 h.

6. Method according to one of Claims 1 to 5, in which the reaction temperature during the use of said lipase extracted from *Geotrichum candidum* is included in the range of from 20 to 40°C.

7. Method according to one of Claims 1 to 6, in which the lipase/substrate ratio in step i) is at least 0.5% w/w.

8. Method according to one of Claims 1 to 7, in which the substrate/alcohol ratio in step i) ranges from 1:4.5 to 1:0.75, the alcohol being added in one or more additions.

9. Method according to one of Claims 1 to 8, in which said lower aliphatic alcohol is methanol.

10. Method according to one of Claims 1 to 9, in which step ii) of separating the mixture M comprises at least one operation chosen from: distillation, centrifugation, decanting, liquid-liquid extraction by means of apolar organic solvents such as hexane or petroleum ether, and chromatographic separation by selective adsorption.

11. Method according to one of Claims 1 to 10, in which said fraction C enriched with RA esters is subjected to a succession of chemical reactions, producing 11-aminoundecanoic acid.

12. Method according to one of Claims 1 to 10, in which said fraction B enriched with esters of fatty acids other than RA is subjected to a succession of chemical and/or physical reactions, producing biodiesel.

13. Method according to one of Claims 1 to 10, in which the starting material being an oil with a low ricinoleic acid content, said fraction enriched with RA ester contains at least 75% by weight of ricinoleic acid methyl ester.

14. Method for producing 11-aminoundecanoic acid, comprising a method for producing ricinoleic acid ester according to Claim 13 as first step.
